# EUROPÄISCHE PATENTANMELDUNG

(11) **EP 0 964 096 A2**
(43) Veröffentlichungstag der Anmeldung: **15.12.1999**
(21) Anmeldenummer: 99810477.2
(22) Anmeldetag: 02.06.1999
(51) Int. Cl.: D06P 5/06, D06P 1/642, C07D 251/24

(54) **Verfahren zur Verbesserung der photochemischen und thermischen Stabilität von Färbungen und Drucken auf Polyesterfasermaterialien**

(30) Priorität: 11.06.1998 EP 98810533
(71) Anmelder: Ciba Specialty Chemicals Holding Inc., 4057 Basel (CH)
(72) Erfinder: Adam, Jean-Marie, 68300 Rosenau (FR); Bacher, Jean-Pierre, 68220 Buschwiller (FR)

(57) **Zusammenfassung**

Beschrieben wird ein Verfahren zur photochemischen und thermischen Stabilisierung von Färbungen und Drucken auf Polyesterfasermaterialien, welches dadurch gekennzeichnet ist, dass man die Polyesterfasermaterialien mit einer Verbindung der Formel 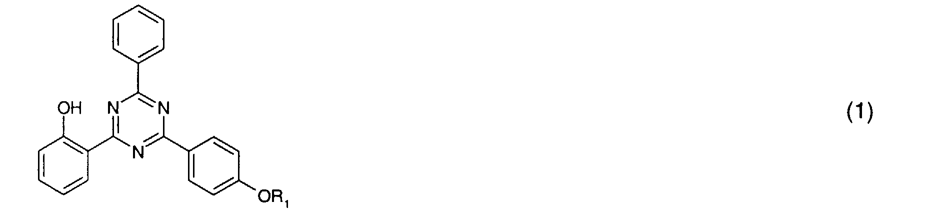 worin R₁ C₁-C₄-Alkyl ist, behandelt.

Die behandelten gefärbten oder bedruckten Polyesterfasermaterialien zeichnen sich durch eine gute Licht- und Wärmeechtheit aus.

## Beschreibung

Die vorliegende Erfindung betrifft ein Verfahren zur photochemischen und thermischen Stabilisierung von Färbungen und Drucken auf Polyesterfasermaterialien und polysterfaserenthaltenden Mischfasern.

Gefärbte oder bedruckte Polyesterfasermaterialien können unter Lichteinfluss und insbesondere bei gleichzeitiger Wärmeeinwirkung geschädigt werden. Für den Einsatz im Automotive-Sektor ist beispielsweise ein wirkungsvoller Schutz dieser gefärbten Fasermaerialien vor UV-Strahlung unerlässlich.

Lichtschutzmittel für Polyesterfasermaterialien auf Triazinbasis sind bekannt. Die Schutzwirkung dieser Verbindungen genügt aber den heutigen Anforderungen, insbesondere im Automotive-Sektor, nicht vollumfänglich.

Es besteht daher weiterhin ein Bedarf nach einem besseren Schutz für die Färbungen und Drucke auf Polyesterfasermaterialien.

Es wurde nun gefunden, dass man mit spezifischen Derivaten des 2-(2'-Hydroxyphenyl)-s-triazines einen sehr guten Schutz von Färbungen und Drucken auf Polyesterfasermaterialien erreichen kann.

Gegenstand der vorliegenden Erfindung ist somit ein Verfahren zur photochemischen und thermischen Stabilisierung von Färbungen und Drucken auf Polyesterfasermaterialien, welches dadurch gekennzeichnet ist, dass man die Polyesterfasermaterialien mit einer Verbindung der Formel 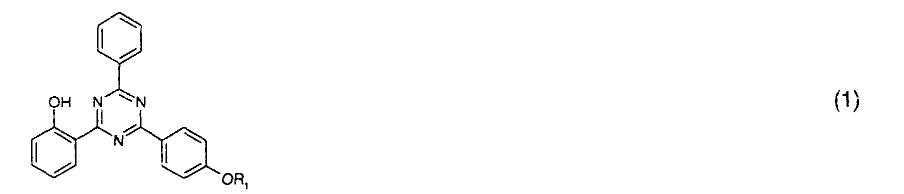 worin
R₁ C₁-C₄-Alkyl ist,
   behandelt.
R₁ als C₁-C₄-Alkyl ist Methyl, Ethyl, Propyl, i-Propyl, n-Butyl, sec.-Butyl, i-Butyl oder tert. Butyl.

Bevorzugt für das erfindungsgemässe Verfahren sind Verbindungen der Formel (1), worin R₁ Ethyl und insbesondere Methyl ist.

Die Verbindungen der Formel (1) sind bekannt.
Die Herstellung der erfindungsgemäss verwendeten Verbindungen der Formel (1) stellt einen weiteren Gegenstand der vorliegenden Erfindung dar. Die Herstellung geschieht z.B. in der Weise, dass man ein o-Hydroxybenzamid der Formel 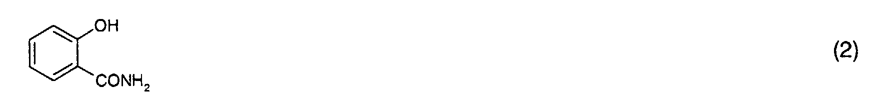 mit einer Verbindung der Formel 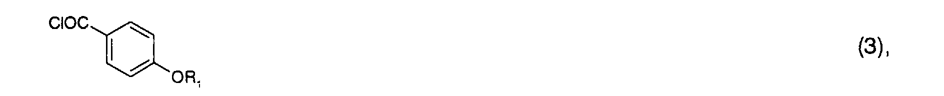 worin
R₁ die unter der Formel (1) angegebene Bedeutung hat,
bei einer Temperatur zwischen 70 und 180° C umsetzt, und das entstandene Zwischenprodukt der Formel 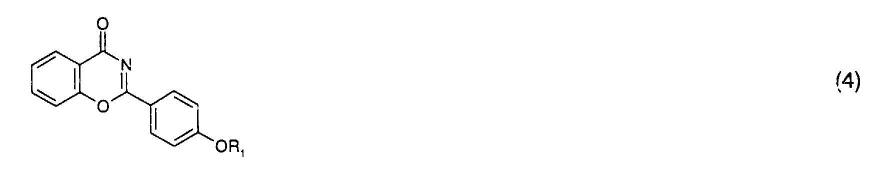 anschliessend mit einer Verbindung der Formel 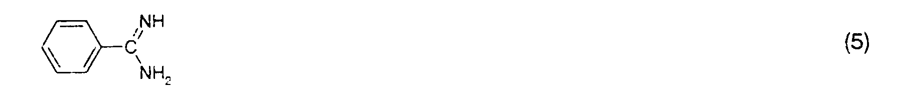 bei einer Temperatur zwischen 50 und 80° C umsetzt. Voerzugsweise werden in dem erfindungsgemässen Verfahren Verbindungen der Formel (3) eingesetzt, worin R₁ Ethyl oder insbesondere Methyl ist.

Die erfindungsgemäss verwendeten Verbindungen der Formel (1) werden in einer Menge von 0,01 bis 5 Gew.-%, vorzugsweise 0,1 bis 3 Gew.-%, bezogen auf das Gewicht des Fasermaterials, eingesetzt.
Die erfindungsgemäss verwendeten Verbindungen sind in Wasser schwerlöslich und werden daher vorteilhafterweise in dispergierter Form appliziert. Dazu werden sie mit einem entsprechenden Dispergator mit Hilfe von z.B. Quarzkugeln und einem Schnellrührgerät auf eine Feinheit von 1-2 mm gemahlen.

Als Dispergatoren für die Verbindungen der Formel (1) kommen z.B. in Betracht:
- saure Ester oder deren Salze von Alkylenoxidaddukten, wie z.B. saure Ester oder deren Salze eines Polyadduktes von 4 bis 40 Mol Ethylenoxid an 1 Mol eines Phenols, oder Phosphorsäureester der Addukte von 6 bis 30 Mol Ethylenoxid an 1 Mol 4-Nonylphenol, 1 Mol Dinonylphenol oder besonders an 1 Mol von Verbin dungen, die durch Anlagerung von 1 bis 3 Mol von gegebenenfalls substituierten Styroien an 1 Mol Phenol hergestellt werden,
- Polystyrolsulfonate,
- Fettsäuretauride,
- alkylierte Diphenyloxid-mono- oder -di-sulfonate,
- Sulfonate von Polycarbonsäureestern,
- mit einer organischen Dicarbonsäure, oder einer anorganischen mehrbasischen Säure in einen sauren Ester übergeführte Anlagerungsprodukte von 1 bis 60, vorzugsweise 2 bis 30 Mol Ethylenoxid und/oder Propylenoxid an Fettamine, Fettamide, Fettsäuren oder Fettalkohole mit je 8 bis 22 Kohlenstoffatomen oder an drei- bis sechs-wertige Alkanole mit 3 bis 6 Kohlenstoffatomen,
- Ligninsulfonate, und ganz besonders
- Formaldehyd-Kondensationsprodukte wie z.B. Kondensationsprodukte von Ligninsulfonaten und/oder Phenol und Formaldehyd, Kondensationsprodukte von Formaldehyd mit aromatischen Sulfonsäuren, wie Kondensationsprodukte von Ditolylethersulfonaten und Formaldehyd, Kondensationsprodukte von Naphthalinsulfonsäure und/oder Naphthol- oder Naphthylaminosulfonsäuren mit Formaldehyd, Kondensationsprodukte von Phenolsulfonsäuren und/oder sulfoniertem Dihydroxydiphenylsulfon und Phenolen bzw. Kresolen mit Formaldehyd und/oder Harnstoff sowie Kondensationsprodukte von Diphenyloxid-disulfonsäurederivaten mit Formaldehyd.

Als Polyesterfasermaterialien, die gefärbt oder bedruckt und mit den Verbindungen der Formel (1) behandelt werden können, sind z.B. Celluloseesterfasern, wie z.B. Cellulose-24-acetatfasern und -triacetatfasern und besonders lineare Polyesterfasern, die eventuell auch sauer modifiziert sind, zu verstehen, die z.B. durch Kondensation von Terephthalsäure mit Ethylenglykol oder von Isophthalsäure oder Terephthalsäure mit 1,4-Bis(hydroxymethyl)-cyclohexan erhalten werden, sowie Fasern aus Mischpolymeren von Terephthal- und Isophthalsäure und Ethylenglykol. Das in der Industrie bisher fast ausschliesslich eingesetzte lineare Polyesterfasermaterial (PES) besteht aus Terephthalsäure und Ethylenglykol.

Die Polyesterfasermaterialien können auch als Mischgewebe unter sich oder mit anderen Fasern, z.B. Mischungen aus Polyacrylnitril/Polyester, Polyamid/Polyester, Polyester/Baumwolle, Polyester/Viskose und Polyester/Wolle, verwendet werden und nach bekannten Verfahren diskontinuierlich oder kontinuierlich gefärbt oder auch bedruckt werden. Die Polyesterfasermaterialien können in verschiedenen Aufmachungsformen vorliegen. Vorzugsweise kommt Stückware, wie Gewirke, Faserverbund oder Gewebe, oder auch Garn auf Kreuzspulen, Kettbäumen usw. in Betracht.

Besonders gut geeignet für das erfindungsgemässe Verfahren sind Polyester-Textilgewebe und polyesterfaserenthaltende Textilmischgewebe, die im Automotive-Sektor eingesetzt werden.

Gut geeignet für das erfindungsgemässe Verfahren sind ferner Polyester-Textilgewebe und Textilmischgewebe enthaltend Polyesterfasern im Oberbekleidungssektor, die lichtdurchlässig sind. Werden solche Textilien nach dem erfindungsgemässen Verfahren behandelt, können sie das unter dem transparenten Oberbekleidungsstoff befindliche Hautgewebe vor dem schädigenden Einfluss der UV-Strahlung schützen.

Wird die erfindungsgemäss verwendete Verbindung der Formel (1) in der Färbeapplikation eingesetzt, so erfolgt die Anwendung z.B. so, dass man das Fasermaterial zunächst mit dieser Verbindung behandelt und anschliessend die Färbung durchführt oder gleichzeitig das Fasermaterial mit einer Verbindung der Formel (1) und dem Farbstoff im Färbebad behandelt. Die Verbindung der Formel (1) kann jedoch auch nachträglich auf die fertig hergestellte Färbung aufgebracht und mittels Thermofixierung, z.B. bei 190 bis 230°C in einem Zeitraum von 30 Sekunden bis 5 Minuten fixiert werden.

Bevorzugt ist die gleichzeitige Applikation der Verbindung der Formel (1) mit einem Farbstoff im Färbebad.

Als für das Färben oder Bedrucken von Polyesterfasermaterialien geeigneten Farbstoffe kommen in Wasser nur gering lösliche Dispersionsfarbstoffe in Betracht.
Es sind z.B. solche Farbstoffe, welche im Colour Index, 3. Auflage (3. Revision 1987 inclusive Additions and Amendments bis No. 85) unter "Disperse Dyes" beschrieben sind.

Die Dispersionsfarbstoffe liegen in der Färbeflotte zum grössten Teil in Form einer feinen Dispersion vor. Sie können verschiedenen Farbstoffklassen angehören, beispielsweise den Amino-, Aminoketon-, Acridon-, Cumarin-, Ketoninim-, Methin-, Perinon-, Naphthochinonimino-, Nitro-, Polymethin-, Diphenylamino-, Chinolin-, Benzimidazol-, Xanthen-, Oxazin-, Chinophthalon-, Styryl-, und insbesondere Anthrachinon- und Azofarbstoffen, wie z.B. Mono- oder Disazofarbstoffen. Es können auch Mischungen von Dispersionsfarbstoffen eingesetzt werden.

Die Färbeflotten können ausser den Farbstoffen und der Verbindung der Formel (1) auch weitere Zusätze, wie z.B.Färbereihilfsmittel, Dispergiermittel, Carrier, Wollschutz- und Netzmittel sowie auch Entschäumer enthalten.

Die Färbebäder können desweiteren Mineralsäuren, wie z.B. Schwefelsäure oder Phosphorsäure, oder zweckmässigerweise organische Säuren, zum Beispiel aliphatische Carbonsäuren wie Ameisensäure, Essigsäure, Oxalsäure oder Zitronensäure und/oder Salze wie Ammoniumacetat, Ammoniumsulfat oder Natriumacetat enthalten. Die Säuren dienen vor allem der Einstellung des pH-Wertes der erfindungsgemäss verwendeten Flotten, der vorzugsweise zwischen 4 und 5 liegt.

Die Färbungen erfolgen aus wässriger Flotte nach einem kontinuierlichen oder diskontinuierlichen Verfahren. Beim diskontinuierlichen Verfahren (Ausziehverfahren) kann das Flottenverhältnis in einem weiten Bereich gewählt werden, z.B. 1:4 bis 1:100, vorzugsweise 1:6 bis 1:50. Die Temperatur, bei der gefärbt wird, beträgt mindestens 50°C und in der Regel ist sie nicht höher als 140°C. Vorzugsweise liegt sie im Bereich von 80 bis 135°C.

Bei kontinuierlichen Färbeverfahren werden die Färbeflotten auf das Stückmaterial durch beispielsweise Foulardieren oder Pflatschen aufgebracht und mittels Thermofixier- oder HT-Dämpfprozessen entwickelt.

Lineare Polyesterfaser und Celluloseesterfaser färbt man vorzugsweise nach dem sogenannten Hochtemperaturverfahren in geschlossenen und druckbeständigen Apparaten bei Temperaturen >100°C, bevorzugt zwischen 110° und 135°C und gegebenenfalls unter Druck. Als geschlossene Gefässe eignen sich beispielsweise Zirkulationsapparaturen, wie Kreuzspul- oder Baumfärbeapparate, Haspelkufen, Düsen- oder Trommelfärbemaschinen, Muff-Färbeapparate, Paddeln oder Jigger.

Cellulose-24-acetatfasern färbt man vorzugsweise bei Temperaturen von 80-85°C.

Vorzugsweise lässt man das Fasermaterial während 5 Minuten bei 40 bis 80°C im Bad, das den Farbstoff, eine Verbindung der Formel (1) und gegebenenfalls weitere Zusätze enthält und auf einen pH-Wert von 4,5 bis 5,5 eingestellt ist, vorlaufen, erhöht die Temperatur innerhalb von 10 bis 20 Minuten auf 125 bis 130°C und behandelt für 15 bis 90 Minuten, vorzugsweise 30 Minuten, bei dieser Temperatur weiter.

Die Fertigstellung der Färbungen erfolgt durch Abkühlen der Färbeflotte auf 50 bis 80°C, Spülen der Färbungen mit Wasser und gegebenenfalls durch Reinigung auf übliche Weise im alkalischen Medium unter reduktiven Bedingungen. Die Färbungen werden dann wiederum gespült und getrocknet.

Wird die Verbindung der Formel (1) vor oder nach der Färbung aus einer separaten Behandlungsflotte auf das Polyesterfasermaterial aufgebracht, so geschieht dies ebenfalls nach einem kontinuierlichen oder diskontinuierlichen Verfahren. Beim diskontinuierlichen Verfahren (Ausziehverfahren) kann das Flottenverhältnis in einem weiten Bereich gewählt werden, z.B. 1:4 bis 1:100, vorzugsweise 1:6 bis 1:50.

Beim kontinuierlichen Verfahren wird die Behandlungsflotte auf das Stückmaterial durch beispielsweise Foulardieren oder Pflatschen aufgebracht und mittels Thermofixier- oder HT-Dämpfprozessen fixiert.

Für die Herstellung von Drucken werden die erfindungsgemäss verwendeten Verbindungen der Formel (1) in Form ihrer wässrigen Dispersionen zweckmässigerweise den Druckpasten beigemischt.
Die Druckpaste enthält dabei die entsprechende Verbindung der Formel (1) in Mengen von 0,01 bis 5 Gew.%, vorzugsweise 0,1 bis 3 Gew.%, bezogen auf das Gewicht der Druckpaste.

Die Verbindungen der Formel (1) können aber bereits bei der Druckbodenvorbereitung, z.B. nach dem Thermofixierverfahren, appliziert werden.

Die Menge der Farbstoffe, die den Druckpasten zugesetzt werden, richtet sich nach der gewünschten Farbnuance; im allgemeinen haben sich Mengen von 0,01 bis 15, vorzugsweise 0,02 bis 10 Gewichtsprozent, bezogen auf das eingesetzte Textilmaterial, bewährt.

Die Druckpasten enthalten neben den Farbstoffen und der wässrigen Dispersion einer Verbindung der Formel (1) zweckmässigerweise säurestabile Verdickungsmittel, vorzugsweise natürlicher Herkunft wie Kernmehlabkömmlinge, insbesondere Natriumalginat für sich allein oder im Gemisch mit modifizierter Cellulose, insbesondere mit vorzugsweise 20 bis 25 Gewichtsprozent Carboxymethylcellulose. Daneben können die Druckpasten noch Säurespender, wie Butyrolacton oder Natriumhydrogenphosphat, Konservie-rungsmittel, Sequestriermittel, Emulgatoren, wasserunlösliche Lösungsmittel, Oxidationsmittel oder Entlüftungsmittel enthalten.
In Betracht kommen als Konservierungsmittel vor allem formaldehydabgebende Mittel, wie z.B. Paraformaldehyd oder Trioxan, vor allem wässrige, etwa 30 bis 40-gewichtsprozentige Formaldehydlösungen; als Sequestriermittel z.B. nitrilotriessigsaures Natrium, ethylendiamintetraessigsaures Natrium, vor allem Natrium-Polymetaphosphat, insbesondere Natrium-Hexamethaphosphat; als Emulgatoren vor allem Addukte aus einem Alkylenoxid und einem Fettalkohol, insbesondere einem Addukt aus Oleylalkohol und Ethylenoxid; als wasserunlösliche Lösungsmittel hochsiedende, gesättigte Kohlenwasserstoffe, vor allem Paraffine mit einem Siedebereich von etwa 160 bis 210°C (sogenannte Lackbenzine); als Oxidationsmittel z.B. eine aromatische Nitroverbindung, vor allem eine aromatische Mono- oder Dinitrocarbonsäure oder -sulfonsäure, die gegebenenfalls als Alkylenoxidaddukt vorliegt, insbesondere eine Nitrobenzolsulfonsäure; und als Entlüftungsmittel z.B. hochsiedende Lösungsmittel, vor allem Terpentinöle, höhere Alkohole, vorzugsweise C₈- bis C₁₀-Alkohole, Terpenalkohole oder Entlüftungsmittel auf Basis von Mineral- und/oder Silikonölen, insbesondere Handelsformulierungen aus etwa 15 bis 25 Gewichtsprozent eines Mineral- und Silikonölgemisches und etwa 75 bis 85 Gewichtsprozent eines C₈-Alkohols wie z.B. 2-Ethyl-n-hexanol.
Beim Bedrucken der Fasermaterialien wird die Druckpaste ganzflächig oder stellenweise direkt auf das Fasermaterial aufgebracht, wobei zweckmässigerweise Druckmaschinen üblicher Bauart, z.B. Tiefdruck-, Rotationssiebdruck-, Flachfilmdruck- oder Inkjetmaschinen eingesetzt werden.

Das Fasermaterial wird nach dem Bedrucken bei Temperaturen bis 150°C, vorzugsweise 80° bis 120°C getrocknet.

Anschliessend erfolgt die Fixierung des Materials durch eine Wärmebehandlung bei Temperaturen von vorzugsweise 100° bis 220°C. Die Wärmebehandlung erfolgt im allgemeinen mit überhitztem Wasserdampf unter Druck.

Je nach Temperatur kann die Fixierung 20 Sekunden bis 12 Minuten, vorzugsweise 4 bis 8 Minuten erfolgen.

Die Fertigstellung der Drucke erfolgt ebenfalls auf übliche Weise durch Spülen mit Wasser und kann gegebenenfalls durch zusätzliche Reinigung im alkalischen Medium unter reduktiven Bedingungen, z.B. mittels Natriumdithionit vorgenommen werden. Im letzteren Fall werden die Drucke wiederum gespült, entwässert und getrocknet.

Mit dem erfindungsgemässen Verfahren lassen sich hochlichtechte und sublimationsbeständige Polyester-Färbungen und Drucke erzielen. Eine gezielte Vor- oder Nachbehandlung des Fasermaterials ist mit dem erfindungsgemässen Verfahren nicht erforderlich.

Einen weiteren Gegenstand der vorliegenden Erfindung stellt die Verwendung der Verbindungen der Formel (1) als Mittel zur photochemischen und thermischen Stabi!isierung von Färbungen und Drucken auf Polyesterfasermaterialien und polyesterfaserenthaltenden Mischfasern dar.

Die nachfolgenden Beispiele dienen der Veranschaulichung der Erfindung. Darin sind die Teile Gewichtsteile und die Prozente Gewichtsprozente. Die Temperaturen sind in Cel-siusgraden angegeben. Die Beziehung zwischen Gewichtsteilen und Volumenteilen ist dieselbe wie diejenige zwischen Gramm und Kubikzentimeter.

### Beispiel 1:

In einer Laborreaktionsapparatur werden
22,86 g p-Anissäure,
75 ml Dioxan, und
0,1 ml Dimethylformamid
vorgelegt. Danach werden innert 10 Minuten
23,2 g Thionylchlorid zugetropft, die Temperatur auf ca. 55° C erhöht und die Reaktionsmischung 90 Minuten bei dieser Temperatur gehalten. Anschliessend wird Dioxan abdestilliert bis zum Erreichen einer Temperatur von 115° C. Danach werden
10,28 g Salicylamid eingetragen und die erhaltene Schmelze 75 Minuten bei 120° C gerührt. Die Reaktionsmischung wird bis ca. 100° C abgekühlt und nach der Zugabe von 70 ml Dioxan so lange gerührt, bis die Temperatur auf 60° C gesunken ist. Danach wird die Reaktionsmischung mit
13 g Benzamidin-chlorhydrat
8,5 g Natriummethylat, und
150 ml Methylalkohol
   versetzt und 30 Minuten bei 60-65° C gerührt. Die entstandene beige Suspension wird abgekühlt, genutscht und mit
175 ml Methylalkohol und
300 ml warmes Wassers bei 45° C gewaschen und im Vakuum bei 50-60° C getrocknet. Man erhält 21.4 g hell-beiges Pulvers der Verbindung der Formel

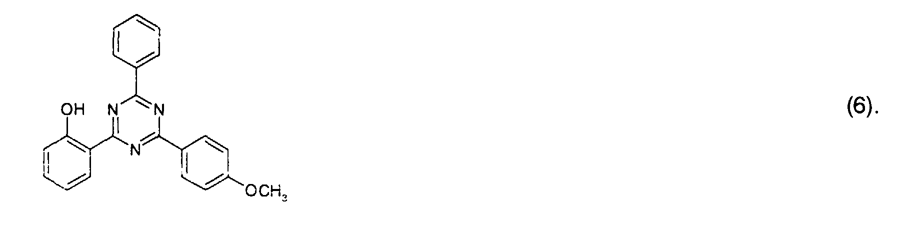

In Analogie zu Beispiel 1 lassen sich die folgenden Verbindungen herstellen, indem man anstelle von p-Anissäure die entsprechenden p-Ethoxybenzoesäure, p-n-Propoxybenzoesäure oder p-lsopropoxybenzoesäure einsetzt:

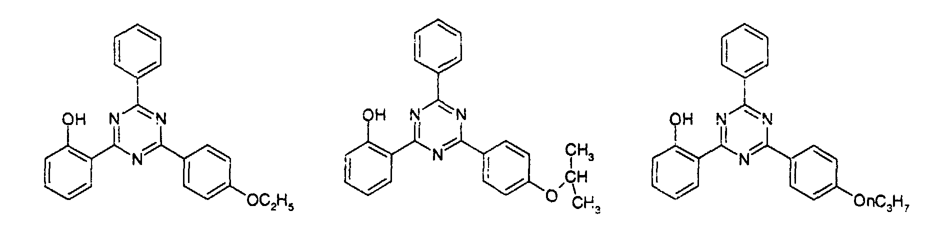

### Beispiel 2:

(a) 5 Gewichtsteile der Verbindung der Forme! (6) gemäss Beispiel 1 werden in einer mit einem Rührer versehenen Laborapparatur mit 2,5 Gewichtsteilen eines Kondensationsproduktes aus Naphthalinsulfonsäure und Formaldehyd als Dispergator, welches in 15 ml Wasser gelöst ist, mit 25 Gewichtsteilen Quarzkügelchen versetzt bei ca. 1600 Umdrehungen/Minute so lange gemahlen, bis eine Teilchengrösse unter 2 um resultiert. Die Dispersion wird anschliessend von den Quarzkügelchen abgetrennt. Durch Zugabe von Wasser wird der Gehalt von der Verbindung der Formel (6) auf 10 Gewichtsprozent, bezogen auf die Formulierung, eingestellt.
(b) Ein Stück von 10 g Polyestertrikot, wird in einer Hochtemperaturfärbeapparatur (Turbomat® der Firma Mathis, Niederhasli, CH) mit einem Flottenverhältnis von 1:10 gefärbt. Die wässrige Färbeflotte enthält pro 1 l der Flotte
   2 g Ammoniumsulfat,
   0,5 g eines handelsüblichen Egalisierhilfsmittels,
   0,5 g der unter (a) hergestellten Formulierung der Verbindung der Formel (6),
   0,83 g einer Farbstoffmischung enthaltend
   0,28 g des Farbstoffes der Formel 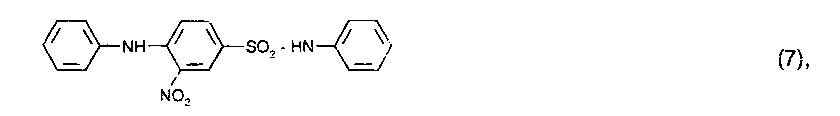
   0,26 g des Farbstoffes der Formel 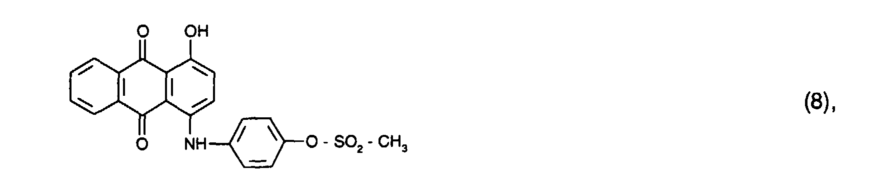
   0,16 g des Farbstoffes der Formel 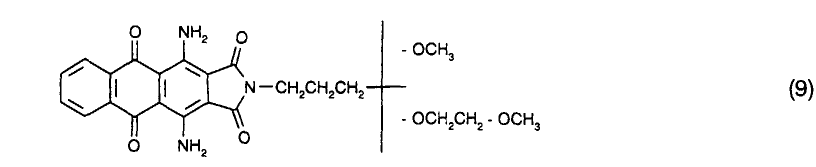 und
   0,13 g des Farbstoffes der Formel

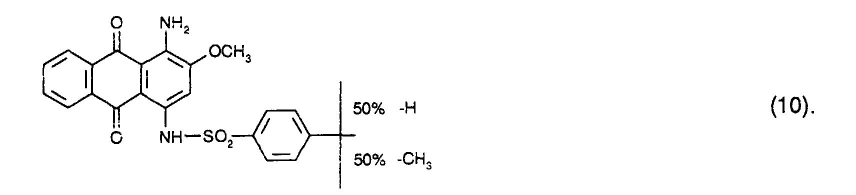

Die Färbeflotte wird mit Essigsäure auf einen pH-Wert von 5 eingestellt, homogenisiert und mit dem Polyestertrikot in die Hochtemperaturfärbeapparatur gegeben und auf 70° C und anschliessend innerhalb von 30 Minuten auf 130° C aufgeheizt. Bei dieser Temperatur wird das Polyestertrikot 60 Minuten gefärbt.
Anschliessend wird die Flotte auf 75° C abgekühlt, das gefärbte Polyestertrikot mit heissem und kaltem Wasser gespült und einer reduktiven Reinigung durch Behandlung mit einer Flotte enthaltend 3 ml/l einer 30%-igen wässrigen Lösung von NaOH und 2 g/l Natriumdithionit, während 20 Minuten bei 70° C unterzogen.
Danach wird das Polyestertrikotstück mit warmem und kaltem Wasser gespült, zentrifugiert und bei 80° C getrocknet.
Man erhält ein grau gefärbtes Polyestertrikot mit guten Allgemeinechtheiten, insbesondere einer sehr guten Heisslichtechtheit.

## Patentansprüche

1. Verfahren zur photochemischen Stabilisierung von Färbungen und Drucken auf Polyesterfasermaterialien, dadurch gekennzeichnet, dass man die Polyesterfasermaterialien mit einer Verbindung der Formel 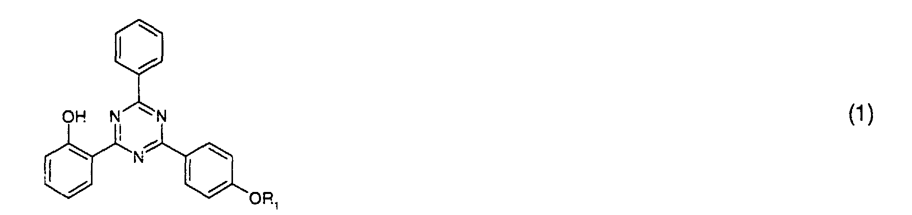 worin R₁ C₁-C₄-Alkyl ist, behandelt.

2. Verfahren gemäss Anspruch 1, dadurch gekennzeichnet, dass man die Polyesterfasermaterialien mit einer Verbindung der Formel (1), worin R₁ Ethyl oder Methyl ist, behandelt.

3. Verfahren gemäss Anspruch 2, dadurch gekennzeichnet, dass man die Polyesterfasermaterialien mit einer Verbindung der Formel (1), worin R₁ Methyl ist, behandelt.

4. Verfahren gemäss Anspruch1, dadurch gekennzeichnet, dass man 0,01 bis 5 Gew.-%, bezogen auf das Gewicht des Fasermaterials, einer Verbindung der Formel (1) verwendet.

5. Verwendung von Verbindungen der Formel (1) gemäss Anspruch 1 zur photochemischen und thermischen Stabilisierung von Färbungen und Drucken auf Polyesterfasermaterialien und polyesterfaserenthaltenden Mischfasern.

6. Verfahren zur Herstellung von Verbindungen der Formel 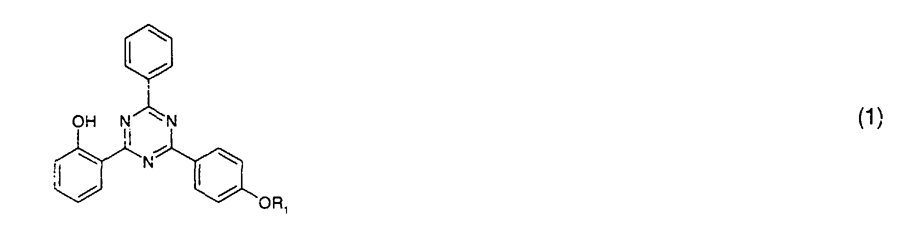 worin R₁ C₁-C₄-Alkyl ist, dadurch gekennzeichnet, dass man ein o-Hydroxybenzamid der Formel 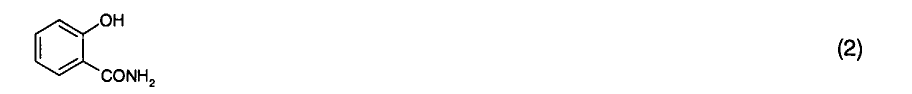 mit einer Verbindung der Formel 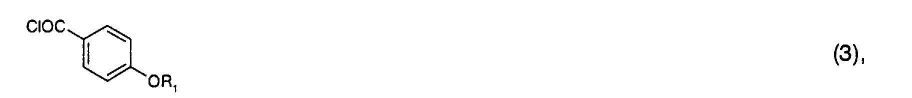 worin
R₁ die unter der Formel (1) angegebene Bedeutung hat,
bei einer Temperatur zwischen 70 und 180° C umsetzt, und das entstandene Zwischenprodukt der Formel 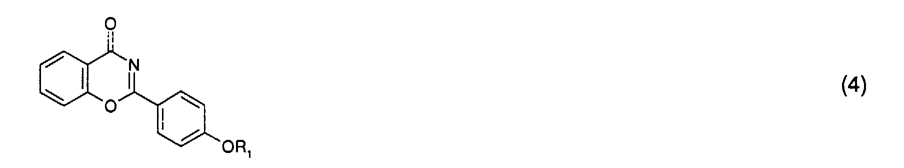 anschliessend mit einer Verbindung der Formel 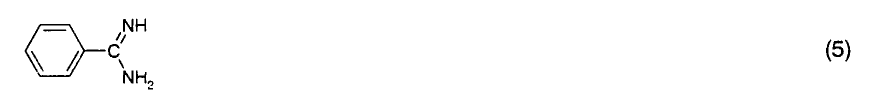 bei einer Temperatur zwischen 50 und 80° C umsetzt.

7. Verfahren gemäss Anspruch 6, dadurch gekennzeichnet, dass man eine Verbindung der Formel (4) einsetzt, worin R₁ Ethyl oder vorzugsweise Methyl ist.
